# EUROPÄISCHE PATENTSCHRIFT

(11) **EP 0 444 381 B1**
(45) Veröffentlichungstag und Bekanntmachung des Hinweises auf die Patenterteilung: **21.07.1993**
(21) Anmeldenummer: 90810950.7
(22) Anmeldetag: 05.12.1990
(51) Int. Cl.: A61F 2/34

(54) **Zementfrei einzusetzende metallische Hüftgelenkspfanne**
Metal hip socket which can be fitted without cement
Prothèse d'acétabule métallique destinée à être fixée sans ciment

(30) Priorität: 01.03.1990 CH 642/90
(43) Veröffentlichungstag der Anmeldung: 04.09.1991
(73) Patentinhaber: GEBRÜDER SULZER AKTIENGESELLSCHAFT, CH-8401 Winterthur (CH); PROTEK AG, 3110 Münsingen-Bern (CH)
(72) Erfinder: Wagner, Heinz, Prof. Dr.-med., Orthopädische Kl., W-8501 Schwarzenbruch (DE); Koch, Rudolf, CH-8267 Berlingen (CH); Willi, Roland, CH-8543 Stadel (CH)

(56) Entgegenhaltungen:
- EP-A- 0 298 234
- EP-A- 0 313 762
- US-A- 4 524 467
- US-A- 4 695 282

## Beschreibung

Die Erfindung betrifft eine zementfrei einzusetzende Hüftgelenkpfanne bestehend aus einer metallischen Aussenschale und aus einer metallischen Innenschale, wie sie aus der EP-A-0298234 bekannt ist. Zweischalige Hüftgelenkpfannen sind in CH-A-672 587 und in CH-A-669 904 gezeigt. Die Innenschale kann bei diesen Konstruktionen nach der Primärverankerung der Aussenschale im Knochengewebe in die Aussenschale eingesetzt werden, da sie über eine Schnappverbindung am Rand der Aussenschale einrastet. Dabei muss die Innenschale gleichmässig über den Umfang an ihrem Aussenrand gegen die Bremswirkung der Schnappverbindung in die Aussenschale eintauchen. Ein verkanten der Innenschale und eine asymmetrische Belastung der Aussenschale lassen sich nur begrenzt vermeiden.

Es ist Aufgabe der Erfindung, eine sich selbst zentrierende Schnappverbindung zwischen einer metallischen Innenschale und einer metallischen Aussenschale herzustellen, die unabhängig vom Kraftangriffspunkt einer Fügekraft auf die Innenschale einrastet und axial fixiert.

Gemäss der Erfindung wird die Aufgabe dadurch gelöst, dass die Aussenschale an ihrem Pol einen Gewindezapfen aus Kunststoff aufweist, der mit der Aussenschale verschraubt ist und der zur Innenschale hin als Pilz mit elastischem Rand ausgeführt ist, welcher in einer konisch hinterschnittenen Bohrung der Innenschale als Schnappverbindung mit Pressitz einrastbar ist.

Der Vorteil der Erfindung ist darin zu sehen, dass die Innenschale auch bei ungünstigen Montageverhältnissen sicher eingebracht und ohne grossen Kraftaufwand mit der Aussenschale verklinkt werden kann. Eine im Knochengewebe primär verankerte Aussenschale wird in ihrer Verankerung durch das Verklinken der Innenschale nicht beeinträchtigt. Die abhängigen Ansprüche 2 bis 5 beziehen sich auf vorteilhafte Weiterbildungen der Erfindung.

Im folgenden wird die Erfindung anhand von einem Ausführungsbeispiel beschrieben. Es zeigen:
- Fig. 1: Einen Längsschnitt durch eine erfindungsgemässe metallische Hüftgelenkspfanne mit einer Aussenchale und einer Innenschale und
- Fig. 2: einen vergrösserten Ausschnitt gemäss Fig. 1 mit der Geometrie für eine Verklinkung von Innen- und Aussenschale im Polbereich.

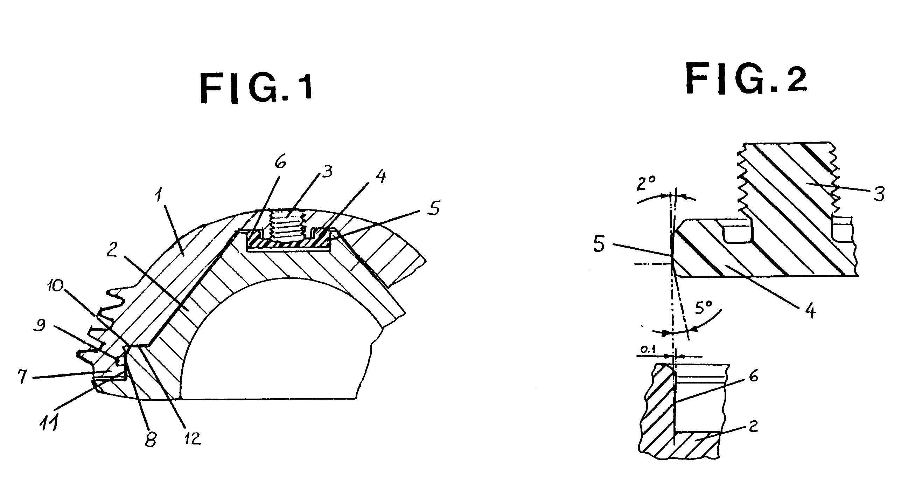

Fig. 1 zeigt in montiertem Zustand eine metallische Aussenschale 1, die vorzugsweise aus Titan besteht, und eine metallische Innenschale 2, die vorzugsweise aus einer Kobaltlegierung besteht. Am Pol der Aussenschale 1 ist auf der Innenseite ein Gewindezapfen 3 aus Kunststoff eingeschraubt, dessen Kopf zur Innenschale 2 hin als Pilz 4 mit elastischem Rand 5 ausgeführt ist. Die Innenschale 2 besitzt am Pol auf ihrer Aussenseite eine konisch hinterschnittene Bohrung 6 mit Untermass gegenüber dem grössten Durchmesser von Pilz 4, in die der Pilz 4 unter axialer Krafteinwirkung einrasten kann. Da der Pilz 4 kleine Abmessungen aufweist und am Grund der Aussenschale 1 liegt, entsteht beim axialen Verklinken aus den Reaktionskräften keine Kippbewegung auf die Aussenschale und wegen des geringen Durchmessers von Pilz 4 ist die Summe der axialen Reaktionskräfte relativ klein.

Damit die mit einer verrundeten Einlaufstrecke versehene Bohrung 6 der Innenschale 2 den konischen Einlauf von Pilz 4 wirklich findet, besitzt die Aussenschale 1 an ihrem Rand 7 zur Innenschale 2 hin eine zylindrische Zentrierfläche 8, in die ein leicht vorstehender Kunststoffring 9 zur Dämpfung eingelassen ist. Die entsprechende Gegenfläche 11 auf der Innenschale 2 verjüngt sich konisch zu einer Schulter 12 hin, um eine einfache Vorzentrierung bei der Montage zu erreichen. Die Eindringtiefe der Innenschale 2 in die Aussenschale 1 wird durch die Schulter 12 bestimmt, die auf einem als Innenschulter ausgebildeten Tiefenanschlag 10 der Aussenschale 1 aufsitzt. Die erste Grobzentrierung findet durch konische Verbindungsflächen statt, die die Aussenschale 1 innen und die Innenschale 2 aussen zwischen Pol und Schalenrand verbinden. Im montierten Zustand fällt das Spiel dieser Flächen zueinander kleiner als ein Millimeter aus, um wenig Gasvolumen in den Gelenkbereich hineinzubringen. In diesem Sinn entwickeln der dämpfende Kunststoffring 9 und der Zentrierzapfen 3 auch eine Dichtwirkung, um das restliche Gasvolumen zwischen Aussenschale 1 und Innenschale 2 einzuschliessen.

In Fig. 2 sind die geometrischen Verhältnisse für die Verklinkung näher ausgeführt. Als Referenz für die Gestaltung der Flächen an Pilz 4 und an Bohrung 6 der Innenschale 2 dient der grösste Durchmesser am elastischen Rand 5 von Pilz 4. Ein Schnappeffekt wird dadurch erreicht, dass sich der kleinste Durchmesser von Bohrung 6 und der grösste Durchmesser von Pilz 4 im montierten Zustand gegenseitig überlappen, und dass der kleinste Durchmesser der Bohrung 6 gegenüber dem ungespannten grössten Durchmesser von Pilz 4 ein Untermass von 0,2 mm besitzt. Bis zum Erreichen des Tiefenanschlags 10 gleiten die Flächen aneinander vorbei, wobei sich der Rand 5 von Pilz 4 dem Profil von Bohrung 6 folgend radial nach innen deformiert und nach dem Durchlaufen des kleinsten Durchmessers von Bohrung 6 radial nach aussen zurückspringt. Im ungespannten Zustand bildet der Pilzrand 5 vom grössten Durchmesser zur Aussenschale hin einen Konus mit dem halben Konuswinkel von 2° und zur Innenschale hin einen Konus mit dem halben Konuswinkel von 5°, wobei dieser Konus mit einem Radius in die Stirnfläche von Pilz 4 übergeht. Ebenso weist die Bohrung 6 einen Einlaufradius zu ihrem kleinsten Durchmesser auf. Der Gewindestift 3 mit Pilz 4 und der Kunststoffring 9 sind bevorzugt in Polyäthylen ausgeführt.

## Patentansprüche

1. Zementfrei einzusetzende Hüftgelenkpfanne bestehend aus einer metallischen Aussenschale (1) und aus einer metallischen Innenschale (2), dadurch gekennzeichnet, dass die Aussenschale (1) an ihrem Pol einen Gewindezapfen (3) aus Kunststoff aufweist, der mit der Aussenschale verschraubt ist und der zur Innenschale (2) hin als Pilz (4) mit elastischem Rand (5) ausgeführt ist, welcher in einer konisch hinterschnittenen Bohrung (6) der Innenschale (2) als Schnappverbindung mit Pressitz einrastbar ist.

2. Hüftgelenkschale nach Anspruch 1, dadurch gekennzeichnet, dass der Gewindezapfen (3) aus Polyäthylen ist.

3. Hüftgelenkschale nach Anspruch 1, dadurch gekennzeichnet, dass die Aussenschale (1) aus Titan und die Innenschale (2) aus einer Kobaltlegierung besteht.

4. Hüftgelenkschale nach Anspruch 1, dadurch gekennzeichnet, dass die Aussenschale (1) an ihrem Rand (7) zur Innenschale (2) eine zylindrische Zentrierfläche (8) mit eingelassenem Kunststoffring (9) aufweist und dass die Zentrierfläche (8) durch einen Tiefenanschlag (10) begrenzt ist.

5. Hüftgelenkschale nach Anspruch 4, dadurch gekennzeichnet, dass die Innenschale (2) eine Gegenfläche (11) zur Zentrierfläche (8) aufweist, die mit einer Schulter (12) endet, welche sich auf dem Tiefenanschlag (10) abstützt.

## Claims

1. An acetabulum to be inserted without the use of cement, consisting of a metal outer shell (1) and metal inner shell (2),
**characterised in that** at its peak the outer shell (1) comprises a threaded journal (3) made of plastic, which is screwed to the outer shell and which towards the inner shell (2) is designed as a mushroom (4) having an elastic edge (5), which can engage with force fit in a conically undercut bore (6) of the inner shell (2) as a snap contact.

2. An acetabulum according to Claim 1,
**characterised in that** the threaded journal (3) is made of polyethylene.

3. An acetabulum according to Claim 1,
**characterised in that** the outer shell (1) is made of titanium and the inner shell (2) is made of a cobalt alloy.

4. An acetabulum according to Claim 1,
**characterised in that** at its edge (7) towards the inner shell (2), the outer shell (1) comprises a cylindrical centring surface (8) with an inset plastic ring (9) and in that the centring surface (8) is limited by a vertical stop (10).

5. An acetabulum according to Claim 4,
**characterised in that** the inner shell (2) comprises a counter-surface (11) for the centring surface (8), which ends in a shoulder (12) supported on the vertical stop (10).

## Revendications

1. Cavité cotyloïde s'installant sans ciment et se composant d'une coque métallique extérieure (1) et d'une coque métallique intérieure (2), caractérisée en ce que la coque extérieure (1) comporte au pôle une tige filetée (3) de matière plastique qui se visse sur la coque extérieure et qui est conformée du côté de la coque intérieure (2) en champignon (4) à bord élastique (5) qui s'enclenche de manière à former une liaison à encliquetage avec ajustage serré dans une cavité (6) de la coque intérieure (2) qui comporte une contre-dépouille conique.

2. Cavité cotyloïde selon la revendication 1, caractérisée en ce que la tige filetée (3) est en polyéthylène.

3. Cavité cotyloïde selon la revendication 1, caractérisée en ce que la coque extérieure (1) est en titane et la coque intérieure (2) en alliage de cobalt.

4. Cavité cotyloïde selon la revendication 1, caractérisée en ce que le bord (7) de la coque extérieure (1) comporte du côté de la coque intérieure (2) une surface cylindrique (8) de centrage dans laquelle est inséré un anneau de matière plastique (9) et en ce que la surface de centrage (8) est limitée par une butée de profondeur (10).

5. Cavité cotyloïde selon la revendication 4, caractérisée en ce que la coque intérieure (2) comporte une surface (11) qui est complémentaire de la surface de centrage (8) et qui s'achève par un épaulement (12) qui prend appui contre la butée de profondeur (10).
